# EUROPEAN PATENT APPLICATION

(11) **EP 4 702 946 A1**
(43) Date of publication of application: **04.03.2026**
(21) Application number: 25198094.2
(22) Date of filing: 26.08.2025
(51) Int. Cl.: A61B 34/10

(54) **KNEE PLANNING**

(30) Priority: 30.08.2024 US 202463688944 P
(71) Applicant: Mako Surgical Corp., Weston, FL 33331 (US)
(72) Inventor: ALI, Azhar, WEST ORANGE, 07052 (US); MARCHAND, Robert, WAKEFIELD, 02879 (US); MITTAL, Yogesh, TULSA, 74105 (US); JACOB, Paul, OKLAHOMA CITY, 73131 (US)
(74) Representative: Regimbeau

(57) **Abstract**

Disclosed herein is a method for surgical planning of a knee joint. The method can include determining a joint line obliquity of a knee j oint and a hip-knee-ankle angle of the knee joint. The method can include comparing the joint line obliquity and the hip-knee-ankle angle to a predetermined value, and generating a surgical plan including recommended adjustments of the joint line obliquity and hip-knee-ankle angle of the knee joint if the joint line obliquity and the hip-knee-ankle angle are different from the predetermined value. The joint line obliquity can be any of an extension line joint line obliquity and flexion joint line obliquity.

## Description

### FIELD OF INVENTION

The present invention relates to a surgical planning method and a surgical system for total knee arthroplasty (TKA), and more particularly to a surgical planning method and a surgical system utilizing a classification system based on joint line obliquity (JLO) for patient-specific surgical planning.

### BACKGROUND OF THE INVENTION

Patient-specific surgical planning in TKA presents significant challenges due to the inherent variations in patient anatomy, including differences in bone morphology, joint alignment, and soft tissue properties. These variations impact the ability to achieve and maintain natural knee biomechanics during surgery, which is critical for successful rehabilitation and long-term function. Traditional alignment techniques, such as mechanical alignment, often do not fully account for individual anatomical differences, leading to suboptimal outcomes in terms of knee function and patient satisfaction.

Although there are existing methods to classify patient phenotypes, determining the proper relationship between a patient's specific anatomical features and the surgical corrections needed to achieve optimal knee balancing remains complex. The growing use of kinematic alignment techniques in TKA, which focus on restoring the patient's pre-disease alignment rather than a generalized mechanical axis, underscores the necessity for more precise and straightforward methods to estimate the patient's unique alignment requirements.

Therefore, there is a need for a surgical system and method that can intraoperatively assess the knee joint to ensure accurate bone resections, optimal implant positioning, and balanced soft tissue, ultimately improving patient outcomes.

### BRIEF SUMMARY OF THE INVENTION

According to the invention there is provided a method for surgical planning of a knee joint as defined in the appended claims.

Disclosed herein are methods and systems for knee joint balancing and surgical planning in knee joint replacement surgery. The methods may involve determining anatomical parameters, such as JLO and hip-knee-ankle (HKA) angle, and comparing these values to predetermined reference values. If deviations from the reference values are detected, adjustments to the JLO, HKA, or both can be made. The JLO may be defined as either an extension or flexion joint line obliquity, based on the differences between specific knee joint angles. The reference values may be determined using statistical methods, including linear regression analysis, based on a plurality of knee joint measurements.

Further disclosed are methods for surgical planning, which may include acquiring preoperative imaging data, determining the JLO and HKA angle from this data, and comparing these values to predetermined reference ranges. A surgical plan may then be generated to adjust these parameters as needed, which may involve determining bone resection depths, implant placement, and soft tissue releases to achieve optimal alignment. The methods may also include measuring knee laxity at various angles, deriving reference ranges from multivariate regression analyses, and generating three-dimensional models of the knee joint to visualize planned adjustments.

In an aspect of the present disclosure, a method for knee joint balancing is provided. A method according to this aspect may include the steps of determining a joint line obliquity of a knee joint, determining a hip-knee-ankle angle of the knee joint, comparing the joint line obliquity and the hip-knee-ankle angle to a predetermined value, and adjusting any of the joint line obliquity and the hip-knee-ankle angle of the knee joint if the joint line obliquity and the hip-knee-ankle angle is different from the predetermined value.

Continuing in accordance with this aspect, the step of adjusting any of the joint line obliquity and the hip-knee-ankle angle of the knee joint may include adjusting the joint line obliquity.

Continuing in accordance with this aspect, the step of adjusting any of the joint line obliquity and the hip-knee-ankle angle of the knee joint may include adjusting the hip-knee-ankle angle.

Continuing in accordance with this aspect, the step of adjusting any of the joint line obliquity and the hip-knee-ankle angle of the knee joint may include adjusting the joint line obliquity and the hip-knee-ankle angle.

Continuing in accordance with this aspect, the joint line obliquity may be any of an extension line joint line obliquity and flexion joint line obliquity. The extension joint line obliquity may be defined by a difference between a medial proximal tibial angle of the knee joint and a lateral distal femoral angle of the knee joint. The flexion joint line obliquity may be defined by a difference between a medial proximal tibial angle of the knee joint and a femoral posterior condylar axis angle of the knee joint.

Continuing in accordance with this aspect, the hip-knee-ankle angle may be defined by a difference between a medial proximal tibial angle of the knee joint and a lateral distal femoral angle of the knee joint.

Continuing in accordance with this aspect, the predetermined value may be determined from a plurality of joint line obliquity measurements and a plurality of hip knee angle measurements from a plurality of knee joints. The predetermined value may be determined using a regression analysis of the plurality of joint line obliquity measurements and the plurality hip knee measurements. The regression analysis may be a linear regression analysis. The step of comparing the joint line obliquity and the hip-knee-ankle angle to a predetermined value may include plotting the joint line obliquity and the hip-knee-ankle angle on a graph. A first axis of the graph may represent the joint line obliquity and a second axis of the graph may represent the hip-knee-ankle angle of the knee joint. The graph may include a trend line representing the predetermined value, the trend line may be generated by the regression analysis.

In accordance with another aspect of the present disclosure, a method for surgical planning of a knee joint is provided. A method according to this aspect may include the steps of acquiring imaging data of a patient's knee joint, determining a joint line obliquity of the knee joint from the imaging data, determining a hip-knee-ankle angle of the knee joint from the imaging data, comparing the determined joint line obliquity and hip-knee-ankle angle values to a predetermined reference range for the respective values, and generating a surgical plan than includes adjusting any of the joint line obliquity and hip-knee-ankle angle if the determined joint line obliquity or hip-knee-ankle angle falls outside the predetermined reference range.

Continuing in accordance with this aspect, the imaging data may include at least one of X-ray images, MRI images, and CT scan data of the knee joint.

Continuing in accordance with this aspect, the step of generating the surgical plan may include determining bone resection depths required to adjust and of the joint line obliquity and the hip-knee-ankle angle.

Continuing in accordance with this aspect, the step of generating the surgical plan may include determining a placement and orientation of implants to achieve any of the joint line obliquity and the hip-knee-ankle angle.

Continuing in accordance with this aspect, the step of generating the surgical plan may include determining a soft tissue release of the knee joint to achieve any of the joint line obliquity and the hip-knee-ankle angle.

Continuing in accordance with this aspect, the method may further include a step of measuring knee laxity at a plurality of flexion and extension angles.

Continuing in accordance with this aspect, the predetermined reference range for the joint line obliquity and the hip-knee-ankle angle may be derived from a plurality of patients' knee joint measurements. The predetermined reference range for the joint line obliquity and the hip-knee-ankle angle may be derived from a multivariate regression analysis to correlate the joint line obliquity and the hip-knee-ankle angle.

Continuing in accordance with this aspect, further including a step of generating a three-dimensional model of the knee joint to visualize the planned adjustments of the joint line obliquity and the hip-knee-ankle angle.

### BRIEF DESCRIPTION OF THE DRAWINGS

A more complete appreciation of the subject matter of the present invention and the various advantages thereof can be realized by reference to the following detailed description, in which reference is made to the following accompanying drawings:
FIG. 1 is a schematic drawing showing a knee joint;
FIG. 2 is a schematic drawing showing a femur and a tibia;
FIG. 3 is a graph showing JLO vs. anatomic hip knee ankle (HKA) angle according to an embodiment of the present disclosure;
FIG. 4 is a graph showing extension JLO vs. extension HKA angle according to an embodiment of the present disclosure;
FIG. 5 is a graph showing flexion JLO vs. flexion HKA angle according to an embodiment of the present disclosure;
FIG. 6 is a graph showing bone-to-bone joint distances during intra-operative knee laxity assessments according to an embodiment of the present disclosure;
FIG. 7 is a graph showing extension JLO vs. extension medial laxity according to an embodiment of the present disclosure;
FIG. 8 is a graph showing extension JLO vs. extension lateral laxity according to an embodiment of the present disclosure;
FIG. 9 is a graph showing flexion JLO vs. flexion medial laxity according to an embodiment of the present disclosure;
FIG. 10 is a graph showing flexion JLO vs. flexion lateral laxity according to an embodiment of the present disclosure, and
FIG. 11 is a flowchart showing steps for surgical planning by correlating limb alignment and JLO according to an embodiment of the present disclosure.

### DETAILED DESCRIPTION

Reference will now be made in detail to the various embodiments of the present disclosure illustrated in the accompanying drawings. Wherever possible, the same or like reference numbers will be used throughout the drawings to refer to the same or like features within a different series of numbers (e.g., 100-series, 200-series, etc.). It should be noted that the drawings are in simplified form and are not drawn to precise scale. Additionally, the term "a," as used in the specification, means "at least one." The terminology includes the words above specifically mentioned, derivatives thereof, and words of similar import. Although at least two variations are described herein, other variations may include aspects described herein combined in any suitable manner having combinations of all or some of the aspects described.

As used herein, the terms "HKA angle," "HKA," and "HKA alignment" will be used interchangeably and as such, unless otherwise stated, the explicit use of either term is inclusive of the other term. Similarly, the terms "joint line obliquity" and "joint line orientation" will be used interchangeably and as such, unless otherwise stated, the explicit use of either term is inclusive of the other term.

In describing preferred embodiments of the disclosure, reference will be made to directional nomenclature used in describing the human body. It is noted that this nomenclature is used only for convenience and that it is not intended to be limiting with respect to the scope of the present disclosure. As used herein, when referring to bones or other parts of the body, the term "anterior" means toward the front part of the body or the face, and the term "posterior" means toward the back of the body. The term "medial" means toward the midline of the body, and the term "lateral" means away from the midline of the body. The term "superior" means closer to the head, and the term "inferior" means more distant from the head.

Disclosed herein are methods and systems for utilizing a JLO classification system for patient-specific surgical planning by correlating limb alignment and JLO with the degree of surgical correction required to achieve optimal knee balancing.

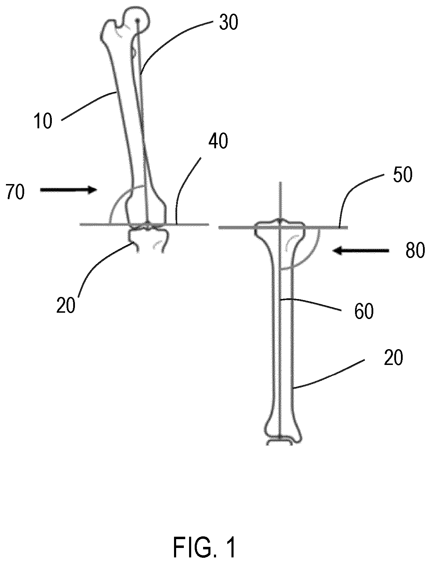

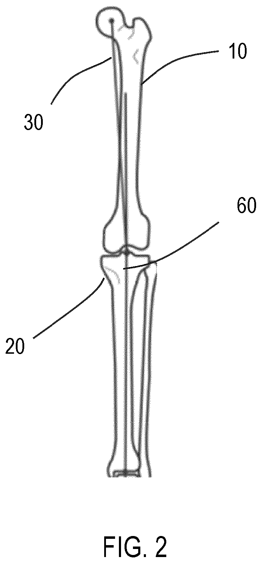

FIGS. 1 and 2 are schematic illustrations of a femur 10 and a tibia 20, demonstrating the measurements utilized in the surgical methods described herein. In FIG. 1, a femoral mechanical axis 30 is shown, extending from the center of the femoral head to the center of the knee joint. Femoral mechanical axis 30 forms a lateral distal femoral angle 70 (LDFA) with a femoral joint line 40, which is one of the angles considered in assessing knee alignment. Similarly, a tibial mechanical axis 60 is depicted, extending from the center of the tibial plateau to the center of the ankle joint. Tibial mechanical axis 60 defines a medial proximal tibial angle 80 (MPTA) with a tibial joint line 50, contributing to the understanding of tibial alignment relative to the knee joint.

HKA angle, which is also referred to as the arithmetic HKA angle, is calculated as the difference between the MPTA and the LDFA. This angle is used in evaluating the overall alignment of the limb and plays a role in planning the surgical approach to achieve the desired knee balance.

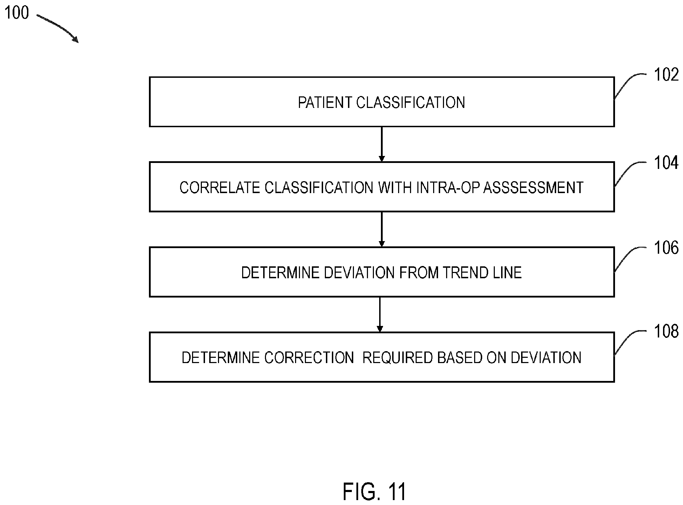

As shown in FIG. 11, a method 100 according to one embodiment of the present disclosure begins with the classification of patients into one of several distinct phenotypes based on measurements of their JLO in a step 102. This classification process can account for the specific measurements of JLO in both extension and flexion, allowing the surgical approach to be tailored to the anatomical characteristics of each individual patient.

Following this classification, a step 104 involves correlating these JLO-based phenotypes with intra-operative assessments of the knee. This step includes evaluating the relationship between the JLO and the HKA angle, using the data obtained during surgery. By analyzing this relationship, the method provides insights that inform the surgical procedure, facilitating adjustments that are aligned with the patient's unique knee alignment profile.

Through the analysis of data collected from multiple patients, a trend line is established that represents the expected relationship between JLO and HKA across different phenotypes. This trend line serves as predictive tool, enabling surgeons or the surgical system to anticipate the specific degree of correction required for an individual patient during surgery in a step 106. This predictive capability is especially valuable in complex cases where achieving the correct alignment and soft tissue balance is challenging.

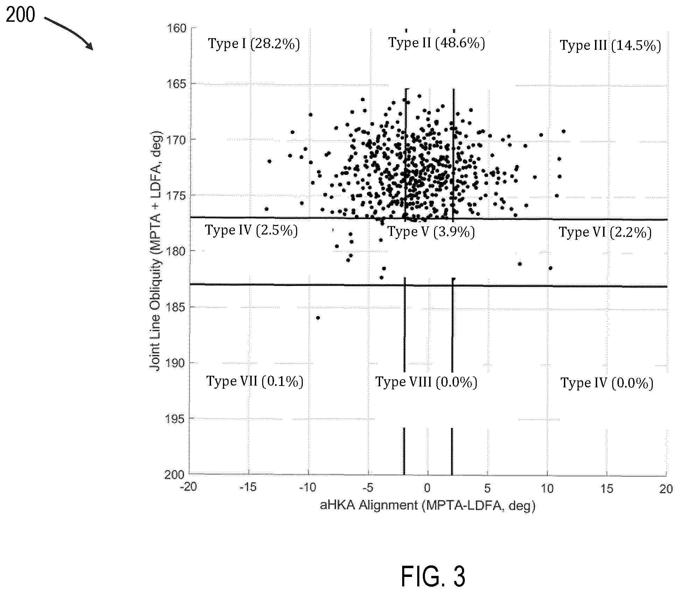

Referring now to FIG. 3, there is shown a graph 200 plotting JLO against the HKA alignment to illustrate the classification of knee phenotypes according to an embodiment of the present disclosure. JLO is defined as the sum of the MPTA the LDFA. As shown in the graph, nine distinct phenotypes are identified based on the relationship between JLO and HKA in this embodiment.

For instance, a Type I phenotype is characterized by an HKA angle of less than -2 degrees, indicating a varus alignment, combined with a JLO greater than 172 degrees. This combination reflects a specific pattern of limb alignment and joint line orientation that is used to guide surgical planning in total knee arthroplasty. Type II, the most common phenotype in the observed population, is associated with a near-neutral HKA and a slightly higher JLO.

Each phenotype occupies a distinct region within the graph, capturing the diversity in knee alignment and offering a visual tool for understanding how different anatomical variations influence surgical outcomes. The classification illustrated in FIG. 3 corresponds to the Coronal Plane Alignment of Knee (CPAK) classification system, as disclosed in U.S. Patent Publication No. 2021/10346036. While this embodiment identifies nine phenotypes, it should be noted that other embodiments may include additional or alternative phenotypes depending on the specific patient population and clinical requirements.

Phenotypic classification provides a systematic approach to categorize patients based on their unique anatomical features. By understanding these phenotypes, surgeons or a surgical system can tailor surgical strategy to address the specific alignment challenges presented by each patient. For example, the identification of a Type V phenotype, characterized by a neutral HKA and a JLO closer to 180 degrees, may suggest a more straightforward surgical intervention with minimal need for extensive correction. Conversely, phenotypes like Type VII, with extreme deviations in both HKA and JLO, may require more complex surgical planning to achieve optimal knee balance.

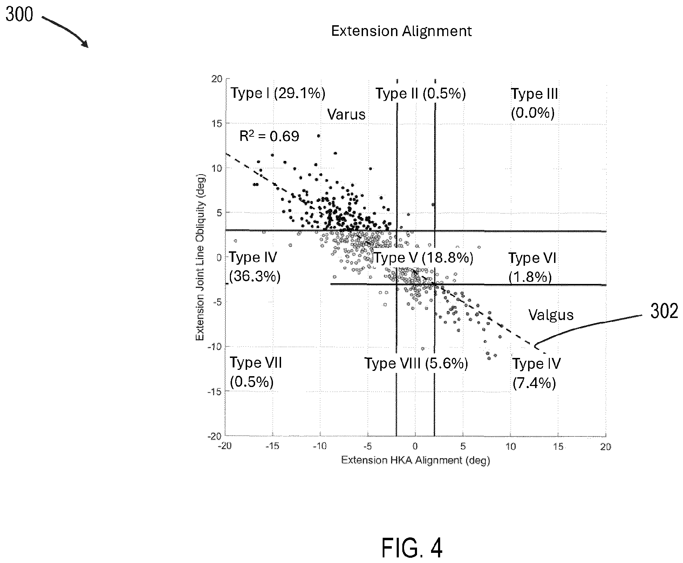

FIG. 4 shows a graph 300 that plots extension JLO against extension HKA alignment according to an embodiment of the present disclosure. Extension JLO is determined by summing the MPTA and the LDFA. Extension HKA alignment is calculated using the varus-valgus angle, which is the angle between the femur and tibial mechanical axes, measured during intra-operative assessment at both 0 degrees (full extension) and 90 degrees (full flexion) without any external force applied to the joint.

As shown in FIG. 4, there is a strong linear relationship between extension JLO and mechanical HKA alignment, as indicated by an R-squared value of 0.69. This strong correlation indicates that the alignment of the joint line is closely related to the overall limb alignment in extension. Such a relationship can be important for understanding how variations in JLO can impact the mechanical alignment of the knee, which in turn affects surgical planning and outcomes in total knee arthroplasty.

This correlation is visualized in graph 300 by a trend line 302, which serves as a predictive tool for determining the degree of correction needed during surgery based on a patient's pre-operative or intra-operative JLO and HKA measurements. For example, a patient with an HKA alignment that deviates significantly from the trend line may require more extensive surgical correction to achieve a well-balanced knee post-operatively.

In addition to trend line 302, graph 300 categorizes patients into specific phenotypes based on their combined JLO and HKA alignment measurements. These phenotypes help to further refine the surgical approach, allowing for more personalized treatment plans. For instance, patients falling into Type IV, which comprises 36.3% of the observed population, typically exhibit a more pronounced varus alignment, indicating a potential need for adjustments in the surgical plan to achieve proper knee balance. Conversely, those in Type V, representing 18.8% of the population, may require less extensive correction due to their closer proximity to neutral alignment.

This data-driven approach enhances the ability to predict surgical outcomes and tailor interventions to the specific anatomical and biomechanical characteristics of each patient. By leveraging the relationship between extension JLO and HKA alignment, surgeons can optimize implant positioning and soft tissue balancing, ultimately contributing to more consistent and favorable patient outcomes in total knee arthroplasty.

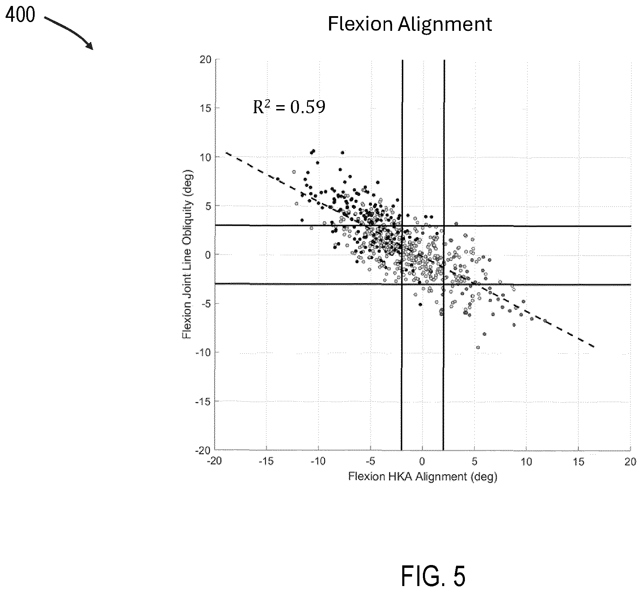

FIG. 5 shows a graph 400 that plots flexion JLO against flexion HKA alignment. In this context, flexion JLO is determined by the relationship between the MPTA and the femoral posterior condylar axis. Flexion HKA alignment is calculated using the varus-valgus angle, which represents the angle between the femur and tibial mechanical axes, measured during intra-operative assessment at both 0 degrees (full extension) and 90 degrees (full flexion) without any external force applied to the joint.

As shown in FIG. 5, there is a notable linear relationship between flexion JLO and flexion HKA alignment, with an R-squared value of 0.59. This indicates a statistically significant correlation, though it is slightly weaker than the correlation observed in extension JLO. Despite the lower R-squared value, the relationship remains important for understanding how variations in JLO during flexion can impact the overall limb alignment.

Graph 400 also features a trend line 402, which can serve as a useful tool for predicting the necessary corrections during surgery. Trend line 402 represents the expected alignment between JLO and HKA for a well-balanced knee. By comparing a patient's measured values to this trend line, surgeons can identify deviations and make targeted adjustments during the procedure to bring the knee alignment closer to the ideal. For example, if a patient's flexion HKA alignment and JLO measurements fall outside the trend line, the surgeon can use this information to modify the surgical approach, such as adjusting the bone cuts, soft tissue adjustment, and/or implant positioning, to achieve better alignment and joint stability. This approach allows for more precise correction of deformities and enhances the potential for successful post-operative outcomes.

Additionally, this data highlights the importance of considering both extension and flexion alignments in surgical planning. While extension measurements may provide a stronger correlation, flexion alignment can be aid in ensuring overall knee balance and function. By integrating both flexion and extension data into the surgical planning process, the method ensures a comprehensive approach to knee alignment, leading to more consistent and favorable patient outcomes.

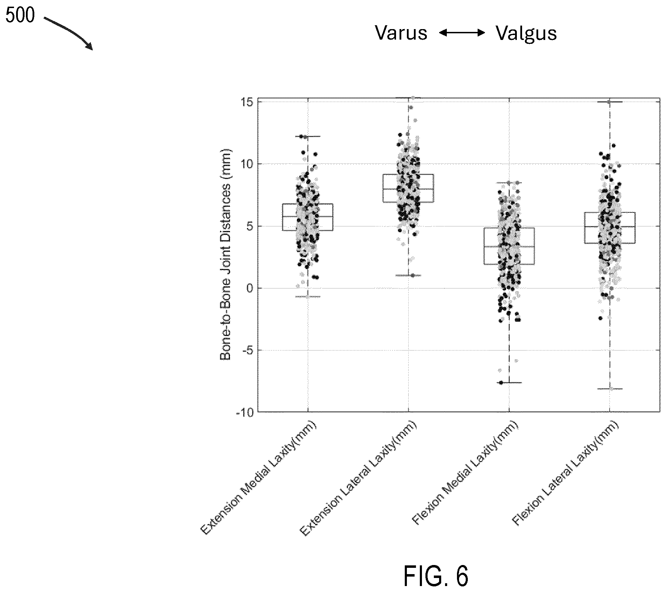

Referring now to FIG. 6, there is shown a graph 500 presenting a box plot illustrating bone-to-bone joint distances measured during intraoperative knee laxity assessments prior to bone resections according to another embodiment. The measurements focus on four distinct types of knee laxity: extension medial laxity, extension lateral laxity, flexion medial laxity, and flexion lateral laxity. These assessments can provide insights into the knee joint's behavior under various conditions of extension and flexion, both medially and laterally.

In this box plot, each laxity measurement is represented by a cluster of data points, with the central box showing the interquartile range, which encompasses the middle 50% of the data. The horizontal line within each box represents the median value for that specific type of laxity, while the whiskers extend to the minimum and maximum values within 1.5 times the interquartile range. Outliers are displayed as individual points beyond the whiskers.

Graph 500 illustrates that the range of bone-to-bone distances varies between the different types of laxity. For example, the flexion lateral laxity measurements tend to show a wider distribution compared to the extension medial laxity, indicating more variability in joint behavior during flexion. Conversely, the extension medial laxity shows a tighter distribution, suggesting more consistent joint behavior during extension in the medial compartment.

These intraoperative measurements aid in understanding the knee's biomechanical properties before making any bone resections. By analyzing these laxity values, surgeons can better tailor their approach to achieve optimal joint balance during total knee arthroplasty. For instance, if a patient exhibits higher medial laxity in extension, the surgeon might consider adjustments in bone resection or implant positioning to achieve a balanced knee post-operatively. Similarly, consistent lateral laxity in flexion could guide the surgeon in fine-tuning the soft tissue tension to ensure stability across the full range of motion. The data represented in this box plot can be used for developing a personalized surgical plan that considers the specific laxity characteristics of each patient's knee.

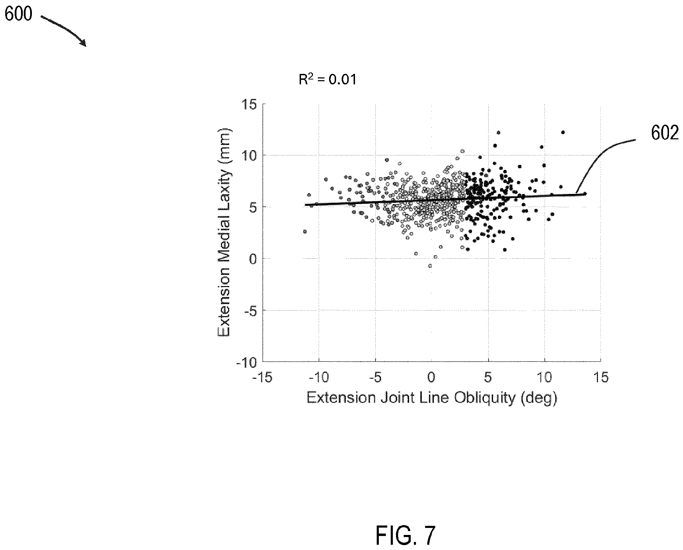

FIGS. 7-10 illustrate the relationship between JLO in both extension and flexion and intraoperative initial knee laxity assessments, according to another embodiment of the present disclosure. These figures provide a detailed comparison of how JLO influences medial and lateral laxity during different phases of knee motion.

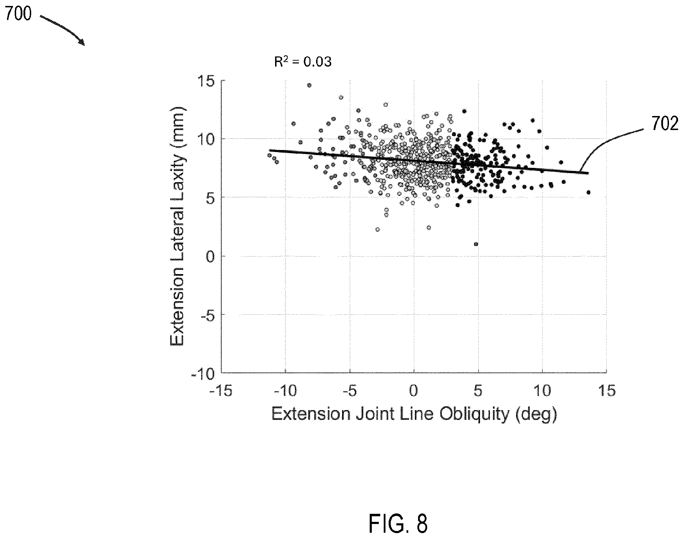

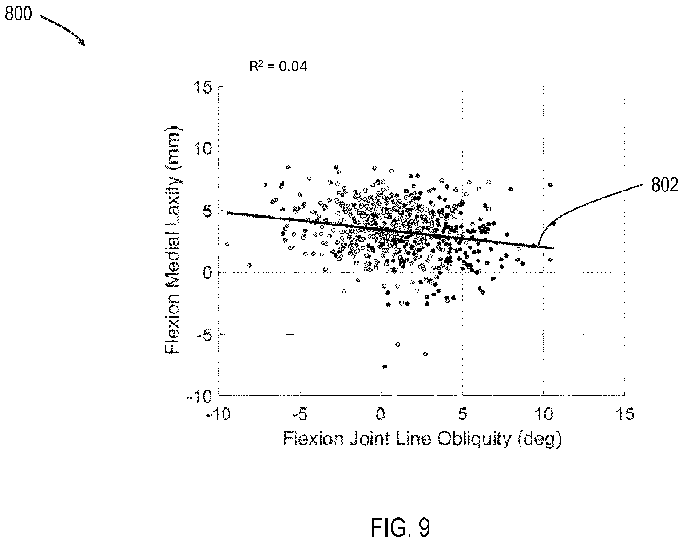

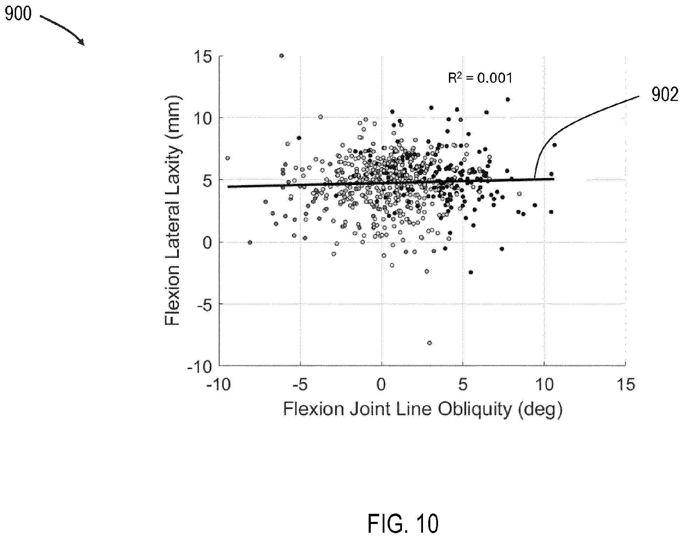

FIG. 7 presents a graph 600 that plots extension medial laxity against extension JLO. FIG. 8 shows a graph 700 that plots extension lateral laxity against extension JLO. FIG. 9 features a graph 800 that plots flexion medial laxity against flexion JLO. FIG. 10 displays a graph 900 that plots flexion lateral laxity against flexion JLO.

In each of these graphs, trend lines (602, 702, 802, 902) are used to visualize the relationships between JLO and knee laxity. The data from FIGS. 7 and 8 indicate that there is a weak correlation between extension JLO and both medial and lateral knee laxity during extension. This weak correlation suggests that the anatomical measurements related to JLO may not be strongly predictive of the initial laxity observed in the medial and lateral compartments during surgery.

Similarly, FIGS. 9 and 10 reveal that the correlation between flexion JLO and both medial and lateral knee laxity during flexion is also weak. Despite the expectation that JLO might influence knee laxity, these findings show that patient phenotypes, as categorized by their JLO measurements, do not appear to have a significant relationship with initial medial or lateral knee laxity.

These observations are significant because they suggest that anatomical factors alone as represented by phenotypes, such as JLO, may not be sufficient to predict soft tissue behavior during knee surgery. Consequently, relying solely on preoperative anatomical classifications may not provide a complete picture for surgical planning. This finding underscores the need of intraoperative balancing assessments, which allow for real-time adjustments based on the actual behavior of the soft tissues, rather than solely on anatomical predictions.

Intraoperative assessments are essential to address variations in initial medial and lateral laxity, ensuring that each patient's knee is balanced and stable following the procedure. By incorporating these assessments into the surgical workflow, surgeons can make more informed decisions that account for individual differences in soft tissue behavior, leading to better outcomes in total knee arthroplasty.

The methods and systems disclosed herein can be implemented either manually or with the assistance of a computerized system, including robotic technology. In a robotic-assisted procedure, for instance, a patient-specific 3D CT-based surgical plan can be generated prior to surgery. This plan is designed to optimize the placement of the implant across the sagittal, transverse, and coronal planes, ensuring precise alignment that is tailored to the patient's unique anatomical features. During intraoperative planning, the tension of the ligaments can be assessed objectively using specialized tensioners. This assessment allows the surgeon to evaluate the balance between the medial and lateral compartments of the knee. Based on this evaluation, the position of the implant can be refined to better accommodate the patient's soft tissue characteristics, ensuring that the knee joint is properly balanced after the procedure.

Once the optimal implant position has been determined, the patient's customized surgical plan can be executed using a robotic system. The robot is programmed to perform the necessary bone resections with high precision, following the predefined plan. The use of robotic assistance enhances the accuracy of the resections and implant placement, reducing the margin for error and improving overall surgical outcomes.

Furthermore, although the invention disclosed herein has been described with reference to particular features, it is to be understood that these features are merely illustrative of the principles and applications of the present invention. It is therefore to be understood that numerous modifications, including changes in the sizes of the various features described herein, may be made to the illustrative embodiments and that other arrangements may be devised without departing from the scope of the present invention. In this regard, the present invention encompasses numerous additional features in addition to those specific features set forth in the paragraphs below. Moreover, the foregoing disclosure should be taken by way of illustration rather than by way of limitation as the present invention is defined in the examples of the numbered paragraphs, which describe features in accordance with various embodiments of the invention, set forth in the paragraphs below.

## Claims

1. A method for surgical planning of a knee joint, the method comprising the steps of:
determining a joint line obliquity of a knee joint from preoperative imaging data;
determining a hip-knee-ankle angle of the knee joint from the preoperative imaging data;
comparing the joint line obliquity and the hip-knee-ankle angle to a predetermined value, and
generating a surgical plan including recommended adjustments to any of the joint line obliquity and the hip-knee-ankle angle of the knee joint if the joint line obliquity and the hip-knee-ankle angle is different from the predetermined value.

2. The method of claim 1, wherein the recommended adjustments include adjusting the joint line obliquity.

3. The method of claim 1, wherein the recommended adjustments include adjusting the hip-knee-ankle angle.

4. The method of claim 1, wherein the recommended adjustments include adjusting the joint line obliquity and the hip-knee-ankle angle.

5. The method of any one of claims 1 to 4, wherein the joint line obliquity is any of an extension line joint line obliquity and flexion joint line obliquity.

6. The method of claim 5, wherein the extension joint line obliquity is defined by a difference between a medial proximal tibial angle of the knee j oint and a lateral distal femoral angle of the knee joint.

7. The method of claim 5 or claim 6, wherein the flexion joint line obliquity is defined by a difference between a medial proximal tibial angle of the knee joint and a femoral posterior condylar axis angle of the knee joint.

8. The method of any one of claims 1 to 7, wherein the hip-knee-ankle angle is defined by a difference between a medial proximal tibial angle of the knee joint and a lateral distal femoral angle of the knee joint.

9. The method of any one of claims 1 to 8, wherein the predetermined value is determined from a plurality of joint line obliquity measurements and a plurality of hip knee angle measurements from a plurality of knee joints.

10. The method of claim 9, wherein the predetermined value is determined using a regression analysis of the plurality of joint line obliquity measurements and the plurality hip knee measurements.

11. The method of claim 10, wherein the regression analysis is a linear regression analysis.

12. The method of claim 10 or claim 11, wherein the step of comparing the joint line obliquity and the hip-knee-ankle angle to a predetermined value includes plotting the joint line obliquity and the hip-knee-ankle angle on a graph.

13. The method of claim 12, wherein a first axis of the graph represents the joint line obliquity and a second axis of the graph represents the hip-knee-ankle angle of the knee joint.

14. The method of claim 13, wherein the graph includes a trend line representing the predetermined value, the trend line being generated by the regression analysis.

15. The method of any one of claims 1 to 14, further comprising generating a three-dimensional model of the knee joint to visualize the recommended adjustments to the joint line obliquity and the hip-knee-ankle angle.
